(19) Europäisches Patentamt
European Patent Office
Office européen des brevets

(11) **EP 4 636 716 A1**

(12) **EUROPEAN PATENT APPLICATION**
published in accordance with Art. 153(4) EPC

(43) Date of publication:
**22.10.2025 Bulletin 2025/43**

(21) Application number: **23902760.0**

(22) Date of filing: **13.12.2023**

(51) International Patent Classification (IPC):
**G06V 40/20** (2022.01)   **G06V 10/774** (2022.01)
**G06V 10/82** (2022.01)   **G06N 3/04** (2023.01)
**G06N 3/084** (2023.01)   **A61B 5/11** (2006.01)

(52) Cooperative Patent Classification (CPC):
**A61B 5/11; G06N 3/04; G06N 3/084; G06V 10/774;
G06V 10/82; G06V 40/20**

(86) International application number:
**PCT/CN2023/138516**

(87) International publication number:
**WO 2024/125566 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.12.2022   CN 202211608610**

(71) Applicant: **Shenzhen Onethird Sleep Technology
Co., Ltd
Shenzhen, Guangdong 518055 (CN)**

(72) Inventors:
• **LIU, Zhong
  Shenzhen, Guangdong 518000 (CN)**
• **LU, Kuntao
  Shenzhen, Guangdong 518000 (CN)**
• **SU, Mingliang
  Shenzhen, Guangdong 518000 (CN)**

(74) Representative: **Cabinet Chaillot
16/20, avenue de l'Agent Sarre
B.P. 74
92703 Colombes Cedex (FR)**

(54) **SLEEPING POSTURE RECOGNITION METHOD AND SYSTEM BASED ON DEEP NEURAL NETWORK**

(57)    Disclosed in the present invention are a sleeping posture recognition method and system based on a deep neural network. The method comprises the steps of: inputting body pressure sample data into a deep neural network for training learning, to obtain a sleeping posture recognition model; obtaining a two-dimensional body pressure array in real time by using a detection device, the detection device obtaining two-dimensional body pressure analog signal data by means of a pressure sensor array, and converting the two-dimensional body pressure analog data into the two-dimensional body pressure array by means of an A/D conversion module; and transmitting the two-dimensional body pressure array to a server for preprocessing, and inputting the pre- processed two-dimensional body pressure array into the sleeping posture recognition model for recognition. According to the present invention, the pressure sensor array is provided at a sleeping position on a mattress to obtain body pressure data in different sleeping postures, and the sleeping posture recognition model is used for performing sleeping posture recognition on the basis of the two-dimensional body pressure array obtained in real time, thereby improving the accuracy of sleeping posture recognition, and solving the problem in the prior art that using a camera to recognize the sleeping posture of a user at night is unlikely to be implemented and invades the privacy of the user.

inputting a body pressure sample data into a deep neural network for training and learning, and obtaining a sleeping posture recognition model — 100

obtaining in real time a two-dimensional array of body pressure using the detection device — 200

transmitting the two-dimensional array of body pressure to a server for preprocessing, inputting the preprocessed two-dimensional array of body pressure into the sleeping posture recognition model for recognition, and outputting a recognition result — 300

FIG. 1

**Description**

## TECHNICAL FIELD

The present invention relates to the technical field of sleeping posture recognition, particularly to a sleeping posture recognition method and system based on deep neural network.

## BACKGROUND

Sleep occupies about one-third of a person's life and significantly impacts work, study, and daily life. Sleep is a fundamental need for life and the foundation for maintaining physical and mental health for humans; maintaining good sleep quality plays an extremely important role in the body's self-repair and growth. Studies have shown that sleeping posture is one of the most important factors determining sleep quality, such as sleep stages and sleep difficulties, and is widely used in the medical diagnosis and treatment of sleep disorders.

In prior art, the usual approach is to obtain sleeping posture photos through a camera and judge different sleeping postures by using algorithms for image recognition.

However, people usually cover themselves with blankets during nighttime sleep, which seriously affects the camera's ability to capture data; moreover, obtaining sleeping postures through cameras seriously violates users' privacy.

## SUMMARY

In the prior art, it is difficult to recognize the user's sleeping posture at night through cameras, which also involves a violation of user privacy.

In response to the above issues, a sleeping posture recognition method and system based on CNN deep network are proposed; a pressure sensor array is deployed in a sleeping position on a mattress to obtain body pressure data of different sleeping postures and, then the body pressure sample data is used for training deep neural network to obtain a sleeping posture recognition model, the real-time obtained two-dimensional array of body pressure is used for recognizing sleeping posture, so that the recognition accuracy of sleeping posture is improved and the problem of difficulty in recognizing the user's sleeping posture at night and violation of the user's privacy by cameras in the prior art is addressed.

In the first aspect, a sleeping posture recognition method based on deep neural network, comprising:

Step 100: inputting the body pressure sample data into a deep neural network for training and learning, and obtaining a sleeping posture recognition model;

Step 200: obtaining in real time a two-dimensional array of body pressure using the detection device, wherein the two-dimensional array of body pressure is detected and obtained by the detection device in a sleeping posture on the mattress, the detection device obtains two-dimensional analog signal data of body pressure through a pressure sensor array, and converts the two-dimensional analog data of body pressure into a two-dimensional array of body pressure through an A/D conversion module;

Step 300: transmitting the two-dimensional array of body pressure to a server for preprocessing, inputting the preprocessed two-dimensional array of body pressure into the sleeping posture recognition model for recognition, and outputting the recognition result.

In the first possible embodiment of the sleeping posture recognition method based on deep neural network according to the first aspect of the present invention, step 100 comprises:

Step 110: the server preprocesses the received two-dimensional array of body pressure and obtains body pressure sample data.

In the second possible embodiment according to the first embodiment in the first aspect of the present invention, step 100 further comprises:

Step 120: setting the number of iterations, weights, and bias values for the deep neural network;

Step 130: inputting the body pressure sample data into the deep neural network and calculating the output error between the expected output and the actual output;

Step 140: comparing the output error with the preset error value and making judgment; and

Step 150: repeating steps 130-140 until the number of iterations is completed or the output error is less than the preset error value, and obtaining the sleeping posture recognition model.

In the third possible embodiment according to the second embodiment in the first aspect of the present invention, step 150 comprises the following steps:

Step 151: obtaining the node error and increment for each node in the deep neural network based on the backpropagation of input body pressure sample data;
updating the weight parameters of each layer node of the deep neural network according to the increment.

In the fourth possible embodiment according to the third embodiment in the first aspect of the present invention, step 200 comprises:

Step 210: deploying a flexible piezoresistive film in a sleeping position on a mattress;
Step 220: placing the transverse and longitudinal electrodes respectively on both sides of the flexible piezoresistive film to form an M × N array electrode;
the flexible piezoresistive film has an area of Hcm × Lcm to cover partial human torso.

In the fifth possible embodiment according to the fourth embodiment in the first aspect of the present invention, step 200 further comprises:

Step 230: converting the detected two-dimensional analog signal data of body pressure into two-dimensional digital signal data of body pressure, and obtaining a two-dimensional array of body pressure;
Step 240: transmitting the two-dimensional array of body pressure to the server.

In the sixth possible embodiment according to the fifth embodiment in the first aspect of the present invention, step 300 comprises the following steps:

Step 310: obtaining the first grayscale value of the sleeping posture image corresponding to the maximum body pressure in the two-dimensional array of body pressure;
Step 320: using equation (1) to:

$$\text{Grayscale element} = \frac{\text{Single grayscale value}}{\text{First grayscale value}} \times 255 \qquad (1)$$

obtain a grayscale matrix to reduce noise in the sleeping posture image, and the single grayscale value is the grayscale value in the sleeping posture image corresponding to the element in the two-dimensional array.

In the second aspect, a sleeping posture recognition system based on deep neural network, using the recognition method described in any one of claims 1-7, comprising:

a server;
a detection device;
the detection device is connected to the server for communication;
the detection device, deployed in the sleeping position on a mattress, is used to detect body pressure data of different sleeping postures, and to convert the body pressure data to obtain a two-dimensional array of body pressure;
the server is used to:

preprocess the received two-dimensional array of body pressure, obtain body pressure sample data, and input the body pressure sample data into a deep neural network for training and learning, obtain a sleeping posture recognition model and use the sleeping posture recognition model to recognize real-time preprocessed body pressure data, and output the recognition result;
the detection device comprises:

a pressure sensor array;
and the pressure sensor array is used to obtain two-dimensional analog signal data of body pressure.

In the first possible embodiment according to the sleeping posture recognition system in the second aspect of the present invention, the detection device further comprises:

an A/D conversion module;
and the A/D conversion module is electrically connected to the pressure sensor array and is used to convert the two-dimensional analog signal data of the body pressure into a two-dimensional array of body pressure.

In the second possible embodiment according to the sleeping posture recognition system based on deep neural network in the second aspect of the present invention, the pressure sensor array comprises:

a flexible piezoresistive film;
a transverse electrode;
a longitudinal electrode;
the transverse and longitudinal electrodes are respectively fitted to both sides of the flexible piezoresistive film, forming an M × N array electrode;
the flexible piezoresistive film has an area of Hcm × Lcm to cover partial human torso.

In the sleeping posture recognition method and system based on deep neural network described in the present invention, a sleeping posture recognition method and system based on CNN deep network are proposed, a pressure sensor array is deployed in a sleeping position on a mattress to obtain body pressure data of different sleeping postures and, then the body pressure sample data is used for training the deep neural network to obtain a sleeping posture recognition model, the real-time obtained two-dimensional array of body pressure is used for recognizing sleeping posture, so that the recognition accuracy of sleeping posture is improved and the problem of difficulty in recognizing the user's sleeping posture at night and violation of the user's privacy by cameras in the prior art is addressed.

## BRIEF DESCRIPTION OF THE DRAWINGS

In order to more clearly illustrate the technical solutions of the embodiments of the present invention, a brief introduction will be given to the accompanying drawings required for the description of the embodiments. Obviously, the accompanying drawings described below are some embodiments of the present invention. Those of ordinary skill in the art can obtain other drawings based on these drawings without creative work.

FIG. 1 is the first schematic diagram of a sleeping posture recognition method based on deep neural network in the present invention;
FIG. 2 is the second schematic diagram of a sleeping posture recognition method based on deep neural network in the present invention;
FIG. 3 is the third schematic diagram of a sleeping posture recognition method based on deep neural network in the present invention;
FIG. 4 is the fourth schematic diagram of a sleeping posture recognition method based on deep neural network in the present invention;
FIG. 5 is the fifth schematic diagram of a sleeping posture recognition method based on deep neural network in the present invention;
FIG. 6 is the sixth schematic diagram of a sleeping posture recognition method based on deep neural network in the present invention;
FIG. 7 is the first schematic diagram of a sleeping posture recognition method based on deep neural network in the present invention;
FIG. 8 is the second schematic diagram of a sleeping posture recognition method based on deep neural network in the present invention;
FIG. 9 is a schematic diagram of the sensor array structure of a sleeping posture recognition method based on deep neural network in the present invention.

## DETAILED DESCRIPTION OF THE EMBODIMENTS

The technical solutions in the embodiments of the present invention will be described clearly and completely in conjunction with the accompanying drawings of the embodiments of the present invention. Obviously, the described embodiments are only a part of the embodiments of the present invention, rather than all the embodiments. Based on the embodiments of the present invention, all other embodiments obtained by those of ordinary skill in the art without creative work shall fall within the protection scope of the present invention.

In the prior art, it is difficult to recognize the user's sleeping posture at night through cameras, and it also involves the infringement upon user privacy.

A sleeping posture recognition method and system based on CNN deep network are proposed to address the above problem.

Embodiments of sleeping posture recognition method

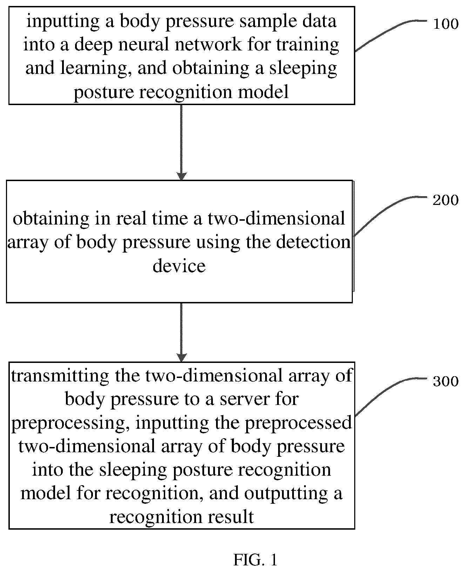

In the first aspect, as shown in Fig. 1, FIG. 1 is the first schematic diagram of a sleeping posture recognition method based on deep neural network in the present invention; a sleeping posture recognition method based on deep neural network, comprising: Step 100: inputting a body pressure sample data into a deep neural network for training and learning, and obtaining a sleeping posture recognition model; Step 200: obtaining in real time a two-dimensional array of body pressure using the detection device 10, wherein the two-dimensional array of body pressure is detected and obtained by the detection device 10 in a sleeping posture on the mattress, the detection device 10 obtains two-dimensional analog signal data of body pressure through a pressure sensor array 11, and converts the two-dimensional analog data of body pressure into a two-dimensional array of body pressure through an A/D conversion module 12; Step 300: transmitting the two-dimensional array of body pressure to a server 20 for preprocessing, inputting the preprocessed two-dimensional array of body pressure into the sleeping posture recognition model for recognition, and outputting the recognition result.

There are mainly 9 types of conventional sleeping postures, including fetal sleeping posture on the left side, fetal sleeping posture on the right side, log sleeping posture on the left side, log sleeping posture on the right side, yearner sleeping posture on the left side, yearner sleeping posture on the right side, soldier sleeping posture, starfish sleeping posture, and freefall sleeping posture.

In the embodiment, a sleeping posture recognition method and system based on CNN deep network are proposed, a pressure sensor array 11 is deployed in a sleeping position on a mattress to obtain body pressure data of different sleeping postures and, then the body pressure sample data is used for training deep neural network to obtain a sleeping posture recognition model, the real-time obtained two-dimensional array of body pressure is used for recognizing sleeping posture, so that the recognition accuracy of sleeping posture is improved and the problem of difficulty in recognizing the user's sleeping posture at night and violation of the user's privacy by cameras in the prior art is addressed.

The pressure sensor array 11 can be a flexible piezoresistive film 112 that is deployed in a person's sleeping position inside the mattress to detect the pressure distribution of sleeping posture, and different sleeping postures have different pressure distributions.

The pressure sensor array 11 converts pressure into electrical signals which are initially analog data of body pressure and the analog data of body pressure is converted into digital signals before further processing.

The pressure sensor array 11 may include M × N array electrodes, and piezoresistive conversion elements disposed within the flexible piezoresistive film 112.

In a preferred embodiment, the M × N array electrodes can be assigned specific values, for example, 256 × 128 array electrodes have 256 transverse electrodes 111 and 128 longitudinal electrodes 113.

Through the A/D conversion module 12, the detected analog data of body pressure is converted into digital signals, and the body pressure data corresponding to a certain sleeping posture is a two-dimensional array of body pressure.

The detection device 10 transmits the two-dimensional array of body pressure to the server 20.

Server 20 also needs to perform image filtering on the images corresponding to the two-dimensional array of body pressure, so as to remove noise from the corresponding images and improve the data accuracy.

In the embodiment, in order to improve the recognition accuracy, the present application constructs a sleeping posture recognition model using preprepared body pressure sample data and deep neural network, and ensures the recognition accuracy through multiple training and learning.

Preferably, step 100 comprises step 110: the server 20 preprocesses the received two-dimensional array of body pressure and obtains body pressure sample data.

Preprocessing is the process where server 20 filters the images corresponding to the received two-dimensional array of body pressure to reduce noise and ensure the accuracy of sample data.

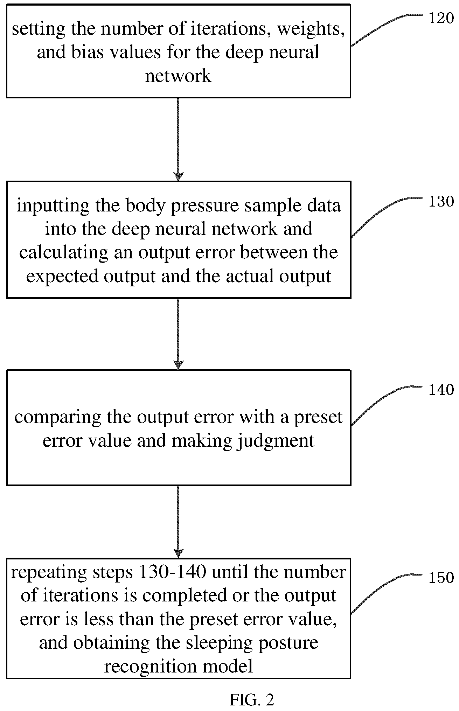

Preferably, as shown in Fig. 2, Fig. 2 is the second schematic diagram of a sleeping posture recognition method based on deep neural network in the present invention; step 100 further comprises step 120: setting the number of iterations, weights, and bias values for the deep neural network; step 130: inputting the body pressure sample data into the deep neural network and calculating the output error between the expected output and the actual output; step 140: comparing the output error with the preset error value and making judgment; and step 150: repeating steps 130-140 until the number of iterations is completed or the output error is less than the preset error value, and obtaining the sleeping posture recognition model.

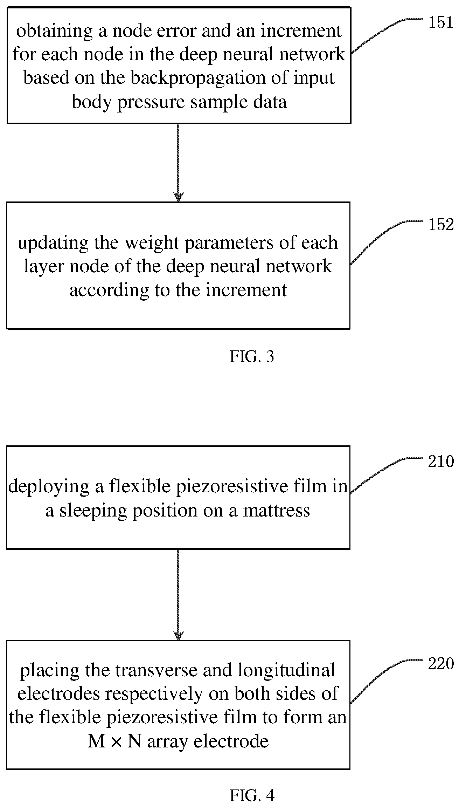

Preferably, as shown in Fig. 3, Fig. 3 is the third schematic diagram of a sleeping posture recognition method based on deep neural network in the present invention; step 150 comprises step 151: obtaining the node error and increment for each node in the deep neural network based on the backpropagation of input body pressure sample data; step 152: updating the weight parameters of each layer node of the deep neural network according to the increment.

Predetermined sample data is used and, after multiple iterations or an error smaller than the preset error is output, a sleeping posture recognition model is obtained.

The larger the weight of signal from a deep neural network, the greater the impact it produces, the neural network stores information in the form of weights, and for the purpose of training, weight parameters need to be updated and, in the embodiment of the application, the sleeping posture recognition model uses backpropagation algorithm to update the weight parameters of deep neural network. Specifically, based on the input sample data of body pressure, backpropagation is used to calculate the error and increment for each node and once the error is obtained, the increment of the node can be calculated, and then equation (2) is used to:

$$\Delta W_{ij} = \alpha I_i x_j$$
$$w_{ij} + \Delta w_{ij} \rightarrow w_{ij} \qquad (2)$$

Update weight parameters;

$w_{ij}$ represents the weight parameter between output node i and input node j, and $\Delta w_{ij}$ is the update amount of weight; $x_j$ is the input sample data of corresponding weight, and a is the bias value (0<a ≤ 1), which determines the change amount of weight each time. The increment $I_i$ of node is obtained from the output error of neural network through reverse calculation.

Preferably, as shown in Fig. 4, Fig. 4 is the fourth schematic diagram of a sleeping posture recognition method based on deep neural network in the present invention; step 200 comprises step 210: deploying a flexible piezoresistive film 112 in a sleeping position on a mattress; step 220: placing the transverse electrode 111 and longitudinal electrode 113 respectively on both sides of the flexible piezoresistive film 112 to form an M × N array electrode; the flexible piezoresistive film 112 has an area of Hcm × Lcm to cover partial human torso.

The pressure sensor array 11 is mainly divided into three layers, with M horizontal electrodes and N vertical electrodes, and piezoresistive films 112 are disposed between the transverse electrode and longitudinal electrode, providing in M × N pressure detection points.

The area of the flexible piezoresistive film 112 of pressure sensor array 11 can be 100cm × 50cm, which is suitable for covering the human torso.

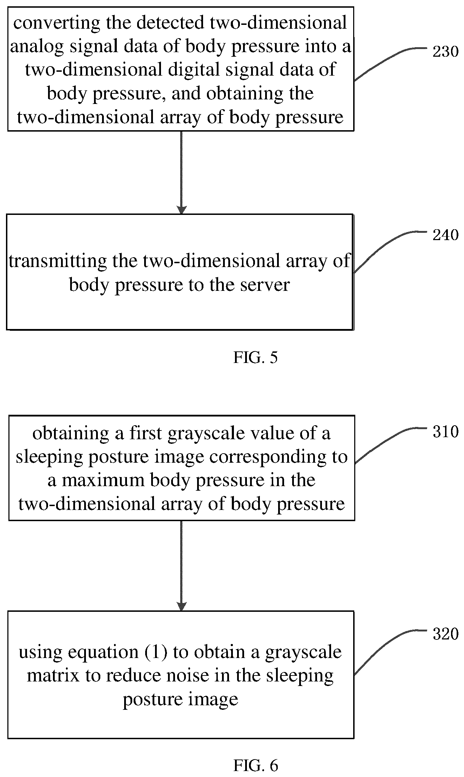

Preferably, as shown in Fig. 5, Fig. 5 is the fifth schematic diagram of a sleeping posture recognition method based on deep neural network in the present invention; step 200 comprises step 230: converting the detected two-dimensional analog signal data of body pressure into two-dimensional digital signal data of body pressure, and obtaining a two-dimensional array of body pressure; step 240: transmitting the two-dimensional array of body pressure to the server 20.

Preferably, as shown in Fig. 6, Fig. 6 is the sixth schematic diagram of a sleeping posture recognition method based on deep neural network in the present invention; step 300 comprises step 310: obtaining the first grayscale value of the sleeping posture image corresponding to the maximum body pressure in the two-dimensional array of body pressure; Step 320: using equation (1) to:

$$\text{Grayscale element} = \frac{\text{Single grayscale value}}{\text{First grayscale value}} \times 255 \qquad (1)$$

obtain a grayscale matrix to reduce noise in the sleeping posture image, and the single grayscale value is the grayscale value in the sleeping posture image corresponding to the element in the two-dimensional array.

Embodiments of sleeping posture recognition system

A sleeping posture recognition system based on deep neural network using the recognition method in the first aspect,

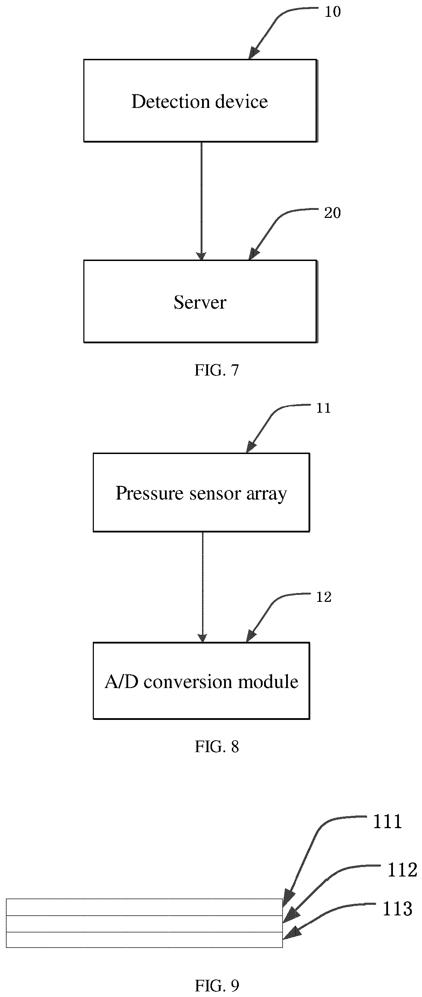

FIG. 7 is the first schematic diagram of a sleeping posture recognition method based on deep neural network in the present invention; comprising a server 20 and a detection device 10; the detection device 10 is connected to the server 20 for communication; the detection device 10, deployed in the sleeping position on a mattress, is used to detect body pressure data of different sleeping postures, and to convert the body pressure data to obtain a two-dimensional array of body pressure; the server 20 is used to preprocess the received two-dimensional array of body pressure, obtain body pressure sample data, and input the body pressure sample data into a deep neural network for training and learning, obtain a sleeping posture recognition model and use the sleeping posture recognition model to recognize the real-time preprocessed body pressure data, and output the recognition result; the detection device 10 comprises a pressure sensor array 11.

First, inputting the body pressure sample data into a deep neural network for training and learning, and obtaining a sleeping posture recognition model; obtaining in real time a two-dimensional array of body pressure using a detection device 10, wherein the two-dimensional array of body pressure is detected and obtained by the detection device 10 in a sleeping posture on the mattress, the detection device 10 obtains two-dimensional analog signal data of body pressure through a pressure sensor array 11, and converts the two-dimensional analog data of body pressure into a two-dimensional array of body pressure through an A/D conversion module 12; then transmitting the two-dimensional array of body pressure to a server 20 for preprocessing, inputting the preprocessed two-dimensional array of body pressure into the sleeping posture recognition model for recognition, and outputting the recognition result.

The pressure sensor array 11 is used to obtain two-dimensional analog signal data of body pressure.

In further, as shown in Fig. 8, Fig. 8 is the second schematic diagram of a sleeping posture recognition method based on deep neural network in the present invention; detection device 10 also includes an A/D conversion module 12; the A/D conversion module 12 is electrically connected to the pressure sensor array 11 to convert the two-dimensional analog data of body pressure into a two-dimensional array of body pressure.

In further, as shown in Fig. 9, Fig. 9 is a schematic diagram of the sensor array structure of a sleeping posture recognition method based on deep neural network in the present invention; the pressure sensor array 11 includes a flexible piezoresistive film 112, a transverse electrode 111, and a longitudinal electrode 113;

The transverse electrode 111 and longitudinal electrode 113 are respectively fitted on both sides of the flexible piezoresistive film 112 to form an M × N array electrode; the flexible piezoresistive film 112 has an area of Hcm × Lcm to cover partial human torso.

The pressure sensor array 11 may include M×N array electrodes, and piezoresistive conversion elements disposed within the flexible piezoresistive film 112. M×N array electrodes can be assigned specific values, for example, 256×128 array electrodes have 256 transverse electrodes 111 and 128 longitudinal electrodes 113.

In the sleeping posture recognition method and system based on deep neural network described in the present invention, a pressure sensor array 11 is deployed in a sleeping position on a mattress to obtain body pressure data of different sleeping postures and, then the body pressure sample data is used for training deep neural network to obtain a sleeping posture recognition model, the real-time obtained two-dimensional array of body pressure is used for recognizing sleeping posture, so that the recognition accuracy of sleeping posture is improved and the problem of difficulty in recognizing the user's sleeping posture at night and violation of the user's privacy by cameras in the prior art is addressed.

Only preferred embodiments of the present invention are described above and it is not intended to limit the present invention. Any modifications, equivalent substitutions, and improvements made within the spirit and principles of the present invention should be included in the scope of protection of the present invention.

## Claims

1. A sleeping posture recognition method based on deep neural network, **characterized in that**, comprising:

   Step 100: inputting a body pressure sample data into a deep neural network for training and learning, and obtaining a sleeping posture recognition model;
   Step 200: obtaining in real time a two-dimensional array of body pressure using the detection device, wherein the two-dimensional array of body pressure is detected and obtained by the detection device in a sleeping posture on the mattress, the detection device obtains a two-dimensional analog signal data of body pressure through a pressure sensor array, and converts the two-dimensional analog data of body pressure into a two-dimensional array of body pressure through an A/D conversion module;
   Step 300: transmitting the two-dimensional array of body pressure to a server for preprocessing, inputting the preprocessed two-dimensional array of body pressure into the sleeping posture recognition model for recognition, and outputting a recognition result.

2. The sleeping posture recognition method based on deep neural network according to claim 1, **characterized in that**, step 100 comprises:
   Step 110: the server preprocesses the received two-dimensional array of body pressure and obtains the body pressure sample data.

3. The sleeping posture recognition method based on deep neural network according to claim 2, **characterized in that**, step 100 further comprises:

   Step 120: setting a number of iterations, weights, and bias values for the deep neural network;
   Step 130: inputting the body pressure sample data into the deep neural network and calculating an output error between the expected output and the actual output;
   Step 140: comparing the output error with a preset error value and making judgment;
   Step 150: repeating steps 130-140 until the number of iterations is completed or the output error is less than the preset error value, and obtaining the sleeping posture recognition model.

4. The sleeping posture recognition method based on deep neural network according to claim 3, **characterized in that**, step 150 comprises the following steps:

Step 151: obtaining a node error and an increment for each node in the deep neural network based on the backpropagation of input body pressure sample data;
Step 152: updating the weight parameters of each layer node of the deep neural network according to the increment.

5. The sleeping posture recognition method based on deep neural network according to claim 4, **characterized in that**, step 200 comprises:

Step 210: deploying a flexible piezoresistive film in a sleeping position on a mattress;
Step 220: placing a transverse electrode and a longitudinal electrode respectively on both sides of the flexible piezoresistive film to form an M $\times$ N array electrode;
wherein the flexible piezoresistive film has an area of Hcm $\times$ Lcm to cover partial human torso.

6. The sleeping posture recognition method based on deep neural network according to claim 5, **characterized in that**, step 200 further comprises:

Step 230: converting the detected two-dimensional analog signal data of body pressure into a two-dimensional digital signal data of body pressure, and obtaining the two-dimensional array of body pressure;
Step 240: transmitting the two-dimensional array of body pressure to the server.

7. The sleeping posture recognition method based on deep neural network according to claim 6, **characterized in that**, step 300 comprises the following steps:

Step 310: obtaining a first grayscale value of a sleeping posture image corresponding to a maximum body pressure in the two-dimensional array of body pressure;
Step 320: using equation (1) to:

$$\text{Grayscale element} = \frac{\text{Single grayscale value}}{\text{First grayscale value}} \times 255 \qquad (1)$$

obtain a grayscale matrix to reduce noise in the sleeping posture image, and the single grayscale value is the grayscale value in the sleeping posture image corresponding to the element in the two-dimensional array.

8. A sleeping posture recognition system based on deep neural network, using the recognition method described in any one of claims 1-7, **characterized in that**, comprising:

a server;
a detection device;
the detection device is connected to the server for communication;
the detection device, deployed in a sleeping position on a mattress, is used to detect a body pressure data of different sleeping postures, and to convert the body pressure data to obtain a two-dimensional array of body pressure;
the server is used to:

preprocess the received two-dimensional array of body pressure, obtain a body pressure sample data, and input the body pressure sample data into a deep neural network for training and learning, obtain a sleeping posture recognition model and use the sleeping posture recognition model to recognize the real-time preprocessed body pressure data, and output a recognition result;
the detection device comprises:

a pressure sensor array;
and the pressure sensor array is used to obtain two-dimensional analog signal data of body pressure.

9. The sleeping posture recognition system based on deep neural network according to claim 8, **characterized in that**, the detection device further comprises:

> an A/D conversion module;
> and the A/D conversion module is electrically connected to the pressure sensor array and is used to convert the two-dimensional analog signal data of the body pressure into the two-dimensional array of body pressure.

10. The sleeping posture recognition system based on deep neural network according to claim 9, **characterized in that**, the pressure sensor array comprises:

> a flexible piezoresistive film;
> a transverse electrode;
> a longitudinal electrode;
> the transverse and longitudinal electrodes are respectively fitted to both sides of the flexible piezoresistive film, forming an M $\times$ N array electrode;
> the flexible piezoresistive film has an area of Hcm $\times$ Lcm to cover partial human torso.

inputting a body pressure sample data into a deep neural network for training and learning, and obtaining a sleeping posture recognition model — 100

obtaining in real time a two-dimensional array of body pressure using the detection device — 200

transmitting the two-dimensional array of body pressure to a server for preprocessing, inputting the preprocessed two-dimensional array of body pressure into the sleeping posture recognition model for recognition, and outputting a recognition result — 300

FIG. 1

setting the number of iterations, weights, and bias values for the deep neural network ⟍ 120

inputting the body pressure sample data into the deep neural network and calculating an output error between the expected output and the actual output ⟍ 130

comparing the output error with a preset error value and making judgment ⟍ 140

repeating steps 130-140 until the number of iterations is completed or the output error is less than the preset error value, and obtaining the sleeping posture recognition model ⟍ 150

FIG. 2

obtaining a node error and an increment for each node in the deep neural network based on the backpropagation of input body pressure sample data — 151

updating the weight parameters of each layer node of the deep neural network according to the increment — 152

FIG. 3

deploying a flexible piezoresistive film in a sleeping position on a mattress — 210

placing the transverse and longitudinal electrodes respectively on both sides of the flexible piezoresistive film to form an M × N array electrode — 220

FIG. 4

converting the detected two-dimensional analog signal data of body pressure into a two-dimensional digital signal data of body pressure, and obtaining the two-dimensional array of body pressure ⎯ 230

transmitting the two-dimensional array of body pressure to the server ⎯ 240

FIG. 5

obtaining a first grayscale value of a sleeping posture image corresponding to a maximum body pressure in the two-dimensional array of body pressure ⎯ 310

using equation (1) to obtain a grayscale matrix to reduce noise in the sleeping posture image ⎯ 320

FIG. 6

Detection device — 10

Server — 20

FIG. 7

Pressure sensor array — 11

A/D conversion module — 12

FIG. 8

111
112
113

FIG. 9

## INTERNATIONAL SEARCH REPORT

International application No.

**PCT/CN2023/138516**

| A. | CLASSIFICATION OF SUBJECT MATTER |
|---|---|

G06V40/20(2022.01)i; G06V10/774(2022.01)i; G06V10/82(2022.01)i; G06N3/04(2023.01)i; G06N3/084(2023.01)i; A61B5/11(2006.01)i

According to International Patent Classification (IPC) or to both national classification and IPC

| B. | FIELDS SEARCHED |
|---|---|

Minimum documentation searched (classification system followed by classification symbols)

IPC: G06V, G06N, A61B

Documentation searched other than minimum documentation to the extent that such documents are included in the fields searched

Electronic data base consulted during the international search (name of data base and, where practicable, search terms used)

CNTXT, CNABS, WPABS, DWPI, ENTXT, IEEE, CNKI: 睡姿, 睡, 姿势, 姿态, 深度, 神经网络, 体压, 床, 压力, 识别, 确定, 判定, sleep, posture, gesture, deep, neural network, pressure, bed, recognition, determine

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115775413 A (SHENZHEN SANFENZHIYI SLEEP TECHNOLOGY CO., LTD.) 10 March 2023 (2023-03-10) entire document | 1-10 |
| X | CN 111353425 A (HEBEI UNIVERSITY OF TECHNOLOGY) 30 June 2020 (2020-06-30) description, paragraphs 0066-0112 | 1, 2, 8-10 |
| Y | CN 111353425 A (HEBEI UNIVERSITY OF TECHNOLOGY) 30 June 2020 (2020-06-30) description, paragraphs 0066-0112 | 3-7 |
| Y | CN 114795129 A (MEIMENG TECHNOLOGY (SHENZHEN) CO., LTD.) 29 July 2022 (2022-07-29) description, paragraphs [0013] and [0014] | 3-7 |
| X | CN 115062704 A (ZHEJIANG SCI-TECH UNIVERSITY) 16 September 2022 (2022-09-16) description, paragraphs 0036-0079 | 1, 2, 8-10 |
| A | CN 113269096 A (ZHEJIANG SCI-TECH UNIVERSITY) 17 August 2021 (2021-08-17) entire document | 1-10 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **18 February 2024** | **22 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/138516** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2021045616 A1 (TECHNISCHE UNIVERSITEIT EINDHOVEN) 11 March 2021 (2021-03-11) entire document | 1-10 |

Form PCT/ISA/210 (second sheet) (July 2022)

**INTERNATIONAL SEARCH REPORT**
Information on patent family members

International application No.

**PCT/CN2023/138516**

| Patent document cited in search report | | | Publication date (day/month/year) | Patent family member(s) | Publication date (day/month/year) |
|---|---|---|---|---|---|
| CN | 115775413 | A | 10 March 2023 | None | |
| CN | 111353425 | A | 30 June 2020 | None | |
| CN | 114795129 | A | 29 July 2022 | None | |
| CN | 115062704 | A | 16 September 2022 | None | |
| CN | 113269096 | A | 17 August 2021 | None | |
| WO | 2021045616 | A1 | 11 March 2021 | None | |

Form PCT/ISA/210 (patent family annex) (July 2022)